# EUROPEAN PATENT APPLICATION

(11) **EP 4 088 651 A1**
(43) Date of publication of application: **16.11.2022**
(21) Application number: 21173562.6
(22) Date of filing: 12.05.2021
(51) Int. Cl.: A61B 5/00, A61B 5/1455, A61B 5/0205

(54) **METHOD FOR DETECTING SLEEP DISORDERING EVENTS**

(71) Applicant: Ectosense NV, 3110 Rotselaar (BE)
(72) Inventor: Massie, Frederik, 3012 Wilsele (BE); Van Pee, Bart, 3000 Leuven (BE); Vits, Steven, 1060 Sint-Gillis (BE)
(74) Representative: IP HILLS NV

(57) **Abstract**

A computer-implemented method for detecting sleep disordering events, the method comprising the steps of obtaining at least one physiological signal; selecting from the at least one physiological signal seed events which are indicatory of sleep disordering events.

## Description

### Field of the Invention

The present invention generally relates to a method for detecting sleep disordering events.

### Background of the Invention

In the field of sleep analysis, the scoring or detection of sleep disordering events, such as obstructive apnea, central apnea, obstructive hypopnea, central hypopnea, or respiratory effort related arousals (RERAs) is generally effected by polysomnography during which different body functions are monitored such as the brain by electroencephalography (EEG), the eye movements by electrooculography (EOG), the muscle activity or skeletal muscle activation by electromyography (EMG), heart rhythm by electrocardiography (ECG), and respiratory airflow. Despite it being considered as the gold standard, this technique has several drawbacks. First, the test itself may require hospitalization or set-up in the home environment by a healthcare professional. Second, the interpretation of the test is often not fully automated, requiring a sleep technician to manually analyse the recorded signals thereby introducing inter-scorer variation. Third, the test may interfere with the person's sleep due to the complex wiring and overall overhead thereby influencing clinical parameters such as supine sleep time, sleep onset, arousals during sleep, and so forth. Fourth, sleeping disorders such as sleep apnea are known to have a high inter-night variability, and the current diagnostic systems are not suited for a multi-night study due the clinical shortcomings, lack of convenience, and high cost per examination.

More recently, the scoring of sleep events has also been performed using a different technique based on measurements by a photoplethysmogram (PPG). PPG measurements at one or more wavelengths allow the monitoring of changes in the peripheral arterial tone, which is indicative of peripheral vasodilation or vasoconstriction, allow deriving the oxygen saturation (SpO₂), and allow deriving the pulse rate. An advantageous embodiment of a system allowing deriving occurrences of sleep events from changes in the peripheral arterial tone (PAT) was for example disclosed in EP 3 593 707 A1. This system, as well as the measurement technique used in the system, allow the detection of sleep disordering events with a minimal impact on, and disturbance of, the sleeping subject, which is a considerable advantage over polysomnography.

Both in polysomnography as well as in a PAT-based approach, the scoring of sleep disordering events is generally based on a co-occurrence of a number of physiological phenomena. In polysomnography, an event may be qualified as a respiratory related sleep disordering event, also called respiratory event, when one or more phenomena co-occur among for example the following: a reduction or even an absence of airflow, an arousal which may be detected by EEG, an oxygen desaturation, detected by a drop in an SpO₂ graph, followed by a return to baseline. For each of these phenomena, thresholds have been determined, for example by the American Academy of Sleep Medicine (AASM), above or below which a measurement may be qualified as being indicative of a respiratory event. In a PAT-based approach as well, co-occurrence of an oxygen desaturation, an increase in heart rate and/or an increase in PAT is considered as being indicative of a respiratory event.

A problem linked with the present approach is that some sleep disordering events may be missed in the scoring process, for example when one of the measurements does not reach a predefined threshold for that feature. Similarly, the scoring process may lead to false positive detections of sleep disordering events. Moreover, home sleep testing devices, such as PAT-based devices or cardiorespiratory polygraphy devices, do not exclusively make use of the gold-standard signals derived from polysomnography, such as airflow or EEG, and therefore, are at an implicit disadvantage in detecting sleep disordering events as they have to infer respiratory events from alternative signal modalities.

It is therefore an aim of the present invention to solve or at least alleviate one or more of the above-mentioned problems. In particular, the invention aims at providing an improved method for detecting sleep disordering events with a relatively high accuracy.

### Summary of the Invention

To this aim, there is provided a computer-implemented method for detecting sleep disordering events characterized by the features of claim 1. In particular, the method comprises the steps of obtaining at least one physiological signal, which signal may include a pointer to a sleep disordering event as is known to the person skilled in the art, identifying seed events indicatory of sleep disordering events from the at least one physiological signal, determining substantially regular patterns within at least part of the seed events, determining degrees of fit of the seed events to the substantially regular patterns, and detecting sleep disordering events by selecting seed events based on the determined degrees of fit. It has been observed that sleep disordering events, which may be respiratory related sleep disordering events such as apnea events, snoring, RERA's, or non-respiratory related sleep disordering events, such as for example spontaneous autonomic arousals, generally occur in groups or sequences at a relatively constant interval. This contextual information is exploited in the present method. By qualifying events as sleep disordering events at least partly based on degrees of fit to substantially regular patterns which are recognized in the data, the accuracy of the detection of sleep disordering events can be increased, since the scoring becomes less dependent on predetermined thresholds than in prior art scoring. In the prior art, sleep disordering events have generally been analysed on a single-event or temporally isolated basis, determining whether or not an individual measurement or two or more co-occurring events in one or more physiological signals were above or below a predetermined threshold to qualify an event as a sleep disordering event without looking at the context of the event. As a result of such a single-event approach, sleep disordering events may have been missed, for example when at least one of the seed events associated with said sleep disordering events was for example just below or above such a threshold. When qualifying the seed events as being associated with sleep disordering or not, the present method does not, contrary to the prior art, simply compare seed events to a predefined threshold. The present method first determines substantially regular patterns in the measured data taking into account an entire data set and then integrates this pattern into the qualification of the seed events as being associated with sleep disordering events or not, which allows to reach a better accuracy in the detection of sleep disordering events than prior art methods.

The at least one physiological signal to be obtained is preferably at least one of a PAT-signal, an oxygen saturation (SpO₂) signal, a pulse rate signal, a respiratory effort signal, and an airflow signal. A PAT-signal or PAT measurement is a measurement of changes in the pulsatile arterial blood volume which reflect changes in arterial tone, and which can for example be measured by mounting a pneumatic or optical sensor at a fingertip of a patient. Oxygen saturation (SpO₂) can be measured by a pulse oximeter, which may be attached to, for example, a patient's finger, nostril, wrist or earlobe. A pulse rate signal may be obtained in many different ways known to the person skilled in the art. A PAT signal, an SpO₂ signal and a pulse rate signal may each be measured by a single device combining said measurements or by separate devices. The devices may be configured to capture directly said signals or to derive said signals from a single measurement. It is preferred to obtain a PAT signal, an SpO₂ signal and a pulse rate signal from a single device configured to perform photoplethysmography (PPG), preferably dual wavelength photoplethysmography, from which a PAT signal, an SpO₂ signal and a pulse rate signal can be derived. Respiratory effort can be derived from, among others, an accelerometer, a bioimpedance measurement, or an inductance plethysmography belt. Airflow can be derived from a measurement by a pressure sensor or an air temperature sensor which may be mounted to a patient's nostril, which is also known to the person skilled in the art. Limb activity can be derived from, among others, an accelerometer, for example attached to an arm or a leg of a patient. Devices which are configured to be attached to a fingertip or a wrist may be preferred in sleep analysis over devices which are attached to the head or chest because the latter devices may hinder natural sleep and thus influence results.

The seed events may for example be at least one of a PAT signal amplitude drop from a baseline, a desaturation of oxygen, a pulse rate signal amplitude increase from a baseline, a respiratory effort signal amplitude decrease from a baseline, an airflow signal amplitude decrease from a baseline, and a limb activity increase from a baseline. These seed events may be indicatory of a sleep disordering event, meaning that they may be a sign or symptom of a sleep disordering event, but are not necessarily associated with a sleep disordering event. In other words, the seed events are events which may, but will not necessarily, be assessed as associated with sleep disordering events. When other physiological signals are obtained, seed events can be defined differently depending on the signal.

The substantially regular patterns can include a substantially regular morphological pattern. A morphological pattern may for example be linked to typical shape features of a representation of a seed event in the obtained signal. As an example, an SpO₂ decrease can show a relatively slow fall and a relatively steep rise to normal. The step of determining the substantially regular patterns and the step of determining degrees of fit can for example include at least one of determining a duration of a seed event, determining an intensity of a seed event, determining a morphological asymmetry of a seed event and/or characterizing a morphological shape of a seed event. A template morphology may for example be derived from a plurality of seed events, neighbouring in time or spaced apart in time. Shape features of a representation of a seed event may then be compared to said template. The step of determining the substantially regular patterns can further include determining features of the substantially regular patterns, for example shape features, which can characterize the substantially regular patterns, for example a shape of said patterns, such as for example an average morphological asymmetry or an average magnitude of the seed events within the substantially regular patterns.

Additionally, or alternatively, the substantially regular patterns can include a substantially regular temporal pattern, such as a repetition of a seed event at predetermined intervals. The step of determining the substantially regular patterns and the step of determining degrees of fit can therefore include at least one of determining a starting point of the seed event, determining an end point of the seed event, determining a point of highest or lowest intensity and/or determining any other characteristic point of a seed event. In this way, a temporal pattern can be recognized in a repetition of seed events, for example in a time difference between starting points of seed events, or between end points of seed events. A temporal distance or time difference between local maxima or minima in the obtained signal may also show a substantially regular temporal pattern. The step of deriving the substantially regular pattern can for example also include determining an interquartile range of a (normalized) difference between neighbouring seed events. The step of determining the substantially regular patterns can further include determining features of the substantially regular pattern which can characterize the substantially regular patterns, for example temporal features, such as for example a number of events in the substantially regular pattern. The step of determining the substantially regular patterns may further include determining a quality of the substantially regular patterns, for example by determining a stability or variability of periodicity between characteristic points of events represented by the substantially regular pattern, for example using a standard deviation or any other known parameter.

It is preferred that the step of detecting sleep disordering events includes using a classifier trained or developed for said detecting. Any conventional classifier, such as a neural network, a decision tree, or a support vector machine, may be trained. The classifier may be trained or developed by designing and inputting a set of rules for said selecting. These rules may for example include rules based on threshold values for one or more measurements. These rules can also include rules based on one or more of the determined values linked to the temporal and/or morphological pattern. As an example, the classifier may use as input, among others, information related to the characterization of the seed events, the degrees of fit of the seed events with the substantially regular patterns, and the characterization of the substantially regular patterns. Alternatively, or additionally, the classifier may also be trained by using labelled data based on previous measurements. A training dataset may for example be constructed from a clinical trial in which by means of manual or computer-aided scoring, the location of sleep disordering events has been scored or annotated and to which seed events can be associated. Alternatively, or additionally, a training dataset may for example be constructed from a clinical trial in which by means of manual or computer-aided scoring, seed events have been labelled as belonging to a sleep disordering event or not.

In a preferred embodiment of the method, a plurality of physiological signals spanning a common time span which includes seed events is obtained. As an example, a PAT signal, an SpO₂ signal and a pulse rate signal can be obtained over a common time span, for example using a single device configured to perform photoplethysmography (PPG), preferably dual wavelength photoplethysmography, from which a PAT signal, an SpO₂ signal and a pulse rate signal can be derived. Alternatively, several measuring devices can be used simultaneously to capture signals indicative of sleep events in a common time span. An accelerometer can for example be attached to a patient's arm or leg while a pressure sensor may be mounted to a patient's nostril. A common time span does not mean that the measurements of a plurality of physiological signals all need to have a same duration or all need to start and stop at the same moment. By 'common time span', it is meant that the physiological signals need to at least partly overlap in time such that they include a time span which is in common or the same for a plurality of said signals and such that said common time span includes seed events. The choice for a given combination of physiological signals may for example be determined by the type of sleep disordering events that need to be distinguished, or by a potential influence of the obtaining of the signal on one's sleep. Many more combinations of physiological signals are possible, as will be clear to the person skilled in the art.

The method can further comprise the step of grouping seed events from the plurality of physiological signals into sets. The grouping can be done such that the seed events grouped into the set are indicatory of the same sleep disordering event. The grouping of seed events into the set can preferably include determining a substantially regular pattern in a co-occurrence of the seed events of the set. As an example, a highest point of a pulse rate spike may for example coincide with a lowest PAT signal amplitude and both events may be preceded by a gradual decrease in peripheral oxygen saturation and be followed by a lowest value in oxygen saturation and a steep rise of oxygen saturation. Time differences between onsets of seed events in different signals may be found to be relatively constant. An order between seed events may slightly vary from person to person but may remain relatively constant for a given patient. Other features may intervene in the step of grouping seed events from the plurality of physiological signals into sets, such as for example a substantial alignment in time of local minima or maxima of physiological signals, in particular a local minimum of oxygen desaturation preceding PAT signal and/or pulse rate local minima or maxima. Another example of a regular pattern in a co-occurrence of seed events may be a detection of a local minimum of a PAT signal and/or pulse rate towards an end of a flow reduction event. Said step of grouping seed events from the plurality of physiological signals into sets may be performed by a classifier trained thereto, or otherwise. Said classifier may be the same type of classifier as the above-mentioned classifier trained to perform the step of detecting the sleep disordering events by selecting seed events based on the determined degrees of fit to substantially regular patterns, which may include substantially regular patterns in a co-occurrence of seed events of a set.

A classifier may advantageously be configured to detect within the sets of seed events the respiratory related sleep disordering events, or the non-respiratory related sleep disordering events. In other words, the classifier may decide that one or more sets of seed events may be associated with respiratory related sleep disordering events while another set of seed events may be associated with non-respiratory related sleep disordering events. In particular, a first classifier may be trained to perform the step of grouping seed events from the plurality of physiological signals into sets, and a second classifier may be trained then to detect from the sets of seed events the sets of seed events associated with respiratory related sleep disordering events, or the sets of seed events associated with non-respiratory related sleep disordering events. The distinction between respiratory related sleep disordering events and non-respiratory related sleep disordering events may for example be based on specific characteristics of the substantially regular pattern and of the set itself. As an example, a periodic limb movement sequence, which is a non-respiratory related sleep disordering event, would not have oxygen desaturations and would have a relatively short time between subsequent events. Alternatively, a single classifier may be trained to perform the step of grouping followed by the step of detecting. A classifier may also be trained to perform the detection step without any grouping of seed events into sets. The classifier may for example have been trained by labelled data sets coming from clinical trials. Alternatively, a classifier may have been trained or developed as a rules-based system based on threshold values of any of the above-mentioned features. The classifier can be any known classifier such as a neural network, a decision tree, or a support vector machine.

The method may further comprise a step of feedback on a detection of a sleep disordering event or not by selection of at least one seed event. The feedback is preferably rendered in natural language. The feedback may for example include information on why a seed event had been assessed as being associated with a sleep disordering event or had been assessed as not being associated with a sleep disordering event. The information may for example be based on a decision tree classifier. The examples given underneath can therefore also be seen as examples of reasoning of the decision tree classifier. The feedback can for example be shown as coded sentences on a screen. A respiratory related sleep disordering event may for example have been detected and the feedback may include information such as "Even though the oxygen desaturation associated with this event is less than 3%, it is part of a train of desaturation events with a relatively large temporal and morphological similarity. The neighbouring two oxygen desaturations, which are part of this train, are on average larger than 3%. The neighbouring six oxygen desaturations, which are a part of this train, are on average larger than 3.5%. The desaturations overlapped largely with trains of pulse rate spikes and PAT reductions with a very similar periodicity" or "The oxygen desaturation associated with this event is part of a train of oxygen desaturation events with a relatively large temporal and morphological similarity. The neighbouring two oxygen desaturations, which are part of this train, are on average larger than 3%. The neighbouring six oxygen desaturations, which are a part of this train, are on average larger than 3.5%. The limb motion surrounding this event was very low". In case a seed event has not been selected or withheld as being associated to a sleep disordering event, the feedback may include information such as "Even though the neighbouring two oxygen desaturations are on average larger than 3% and the neighbouring six oxygen desaturations are on average larger than 3.5%, there was too much limb movement around this location and the surrounding oxygen desaturations had low temporal and morphological similarity". The language or wording of the feedback information need of course not be the same as for the present application. However, it will be clear to the person skilled in the art that the 'temporal and morphological similarity' refer to the degrees of fit to the substantially regular patterns in the seed events. Other ways of rendering this information are possible as well.

### Brief Description of the Drawings

Fig. 1 shows a first schematic graph representing of a plurality of physiological signals over time;
Fig. 2 shows a second schematic graph representing of a plurality of physiological signals over time;
Fig. 3 shows the second schematic graph of Figure 2 illustrating an embodiment of the method according to the present invention;
Fig. 4 shows a third schematic graph representing of a plurality of physiological signals over time;
Fig. 5 shows a fourth schematic graph representing of a plurality of physiological signals over time; and
Fig. 6 shows a computing system suitable for performing various steps according to example embodiments.

### Detailed Description of Embodiment(s)

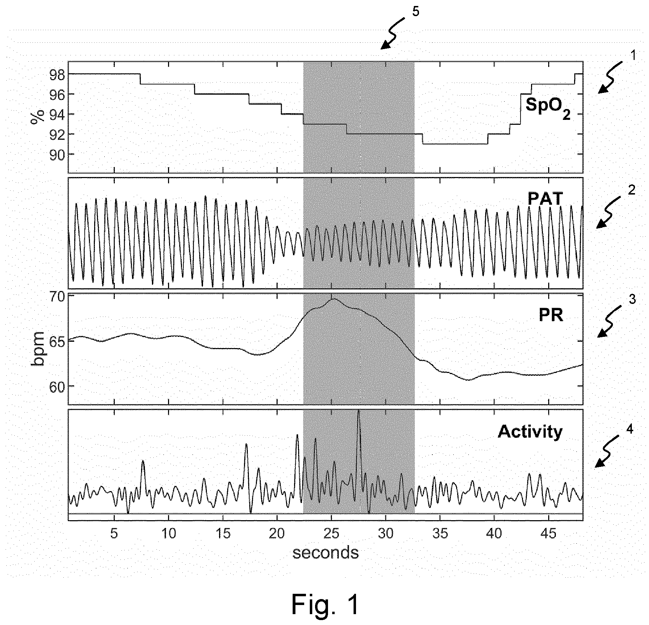

Figure 1 shows a first schematic graph representing of a plurality of physiological signals over time. In the present graph, the physiological signals include a saturation of oxygen (SpO₂) signal 1, a PAT signal 2, a pulse rate signal 3 and a limb activity signal 4. The saturation of oxygen, PAT and pulse rate signals 1,2, 3 may for example be derived from measurements by a single device, for example by measuring arterial pulsatile volume changes at the fingertip, for example by a device as disclosed in EP3,593,707. However, the measurements may also be measured by separate devices. The activity signal 4 may for example be derived from an accelerometer attached to an arm or a leg of a patient. In prior art methods, an amplitude of a drop in SpO₂ as observed in the saturation of oxygen signal 1 would mainly be compared to a threshold value, as set for example by the AASM, to decide whether or not the drop in SpO₂ could be assessed as a sleep disordering event. A similar way of proceeding is used for the drop in PAT amplitude in the PAT signal 2 or for the pulse rate and activity spike in the respective signals 3 and 4. If all or a number of these four indicators point to a potential sleep disordering event, the prior art method would come to the conclusion that a single apneic or sleep disordering event has been detected. In these prior art methods for detecting sleep disordering events, potential indicators of sleep disordering events in physiological signals are mainly treated individually, i.e. compared individually to threshold values without taking into account any context information from the rest of the physiological signal. At most, prior art algorithms combine threshold values of more or less coincidental indicators, as shown in Figure 1, where a co-occurrence 5 of an SpO₂ drop below a predefined threshold, a PAT amplitude drop below a predefined threshold, a pulse rate spike above a predefined threshold and a limb activity spike above a predefined threshold is observed.

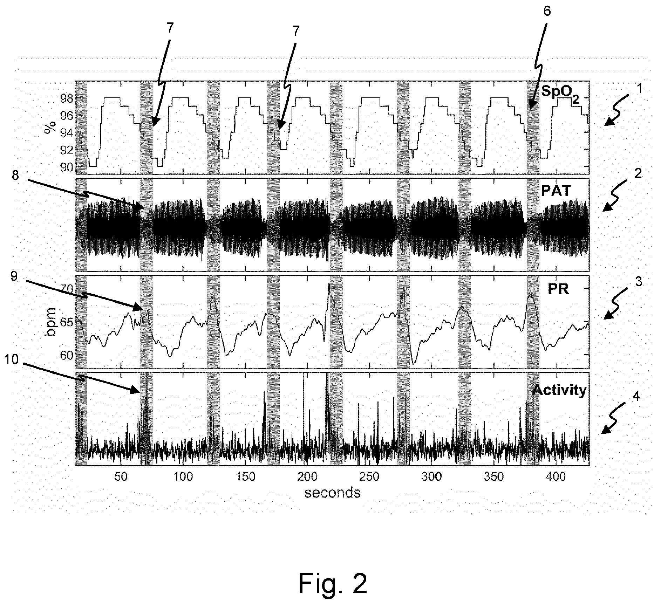

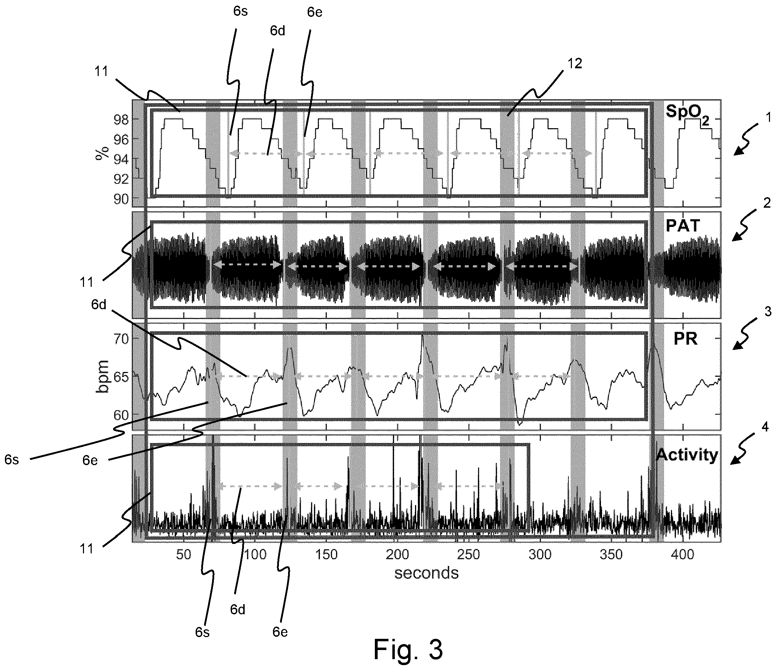

Fig. 2 shows a second schematic graph representing of a plurality of physiological signals spanning a common time span. As in the first graph, the physiological signals include a saturation of oxygen (SpO₂) signal 1, a PAT signal 2, a pulse rate signal 3 and a limb activity signal 4. In a method according to the present invention, it is also possible to obtain only one of these physiological signals, for example only a SpO₂ signal 1 or any combination of at least two of these signals. According to the method, seed events 6, which are indicatory of sleep disordering events, are selected. The seed events 6 may for example be one of a desaturation of oxygen 7, a PAT signal amplitude drop 8 from a baseline, a pulse rate signal amplitude increase 9 from a baseline, a respiratory effort signal amplitude decrease from a baseline, an increase in limb activity 10 or any other known physiological signal which may be a marker of a sleep disordering event. The seed events 6 are only indicatory of a sleep disordering event but need not necessarily point to a sleep disordering event since they may have another underlying cause. In a next step, and contrary to prior art methods, substantially regular patterns will be determined within at least part of the seed events 6 selected in a physiological signal. These substantially regular patterns may include substantially regular morphological patterns, i.e. related to a shape of the representation of the physiological signal, or related to an amplitude of the signal corresponding to an intensity of a seed event, or an inclination, skewness or a morphological asymmetry of an increase or decrease of a seed event in the signal. The determination of substantially regular morphological patterns can thus include a characterization of a morphological shape of a seed event. These substantially regular patterns may also include substantially regular temporal patterns, as will be shown in Figure 3. Such patterns can be especially well recognized for example in the SpO₂ signal 1 and in the PAT signal 2 but are also present in the other physiological signals.

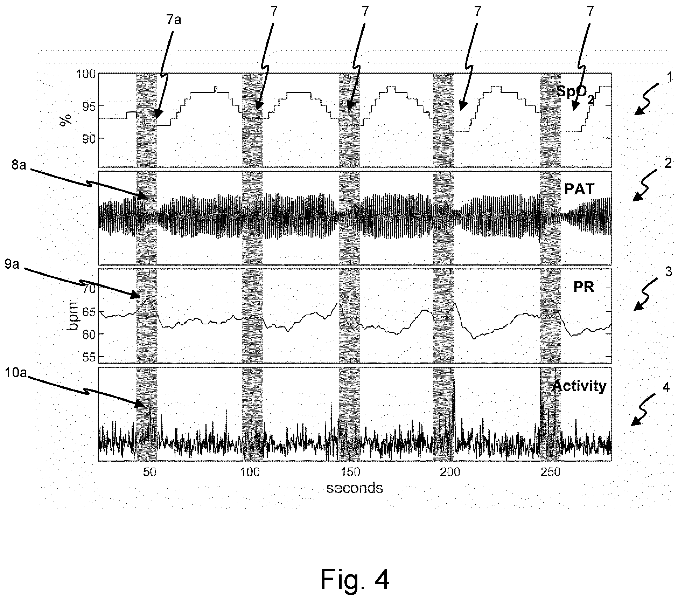

Fig. 3 shows the second schematic graph of Figure 2 illustrating an embodiment of the method according to the present invention. The step of determining substantially regular patterns, for example substantially regular temporal patterns, and the step of determining degrees of fit can for example include determining a duration or time span 6d between subsequent seed events 6, for example by determining a characteristic point 6s, such as a local minimum or maximum of the amplitude of the seed event 6, and determining a subsequent characteristic point 6e of the subsequent seed event. This step may be performed in one or more of the physiological signals separately. By selecting seed events based on the determined degrees of fit with the substantially regular patterns, for example based on a substantially regular duration of seed events in a physiological signal, sleep disordering events can be detected with a relatively high degree of confidence, as will be further explained in Figure 4. Such a detection of sleep disordering events may for example be done by a classifier which has been developed or trained for such detecting. Additionally, and/or alternatively, the step of determining the substantially regular patterns and the step of determining degrees of fit may also include at least one of determining a point of highest or lowest intensity and/or determining any other characteristic point of a seed event. The method can further comprise the step of coupling seed events into sequences or trains of seed events in a physiological signal based on said substantially regular patterns, for example based on similar temporal characteristics, such as a similar duration of the seed event, which step corresponds to exploiting the determination of the degree of fit of a seed event to the substantially regular patterns. As an example, a plurality of seed events in the physiological signals 1, 2, 3 and 4 represented in Figure 3 have been grouped or coupled within the respective physiological signal 1, 2, 3 and 4 based on a similar event duration 6d, as indicated by the coupling rectangles 11. These trains of seed events or coupled seed events may be indicatory of a repetition of a sleep disordering event. Additionally, and/or alternatively, to the coupling of seed events 6 within a physiological signal, the method can further comprise the step of grouping seed events from the plurality of physiological signals into sets 12, the seed events grouped into the set 12 being indicative of the same sleep disordering event. In other words, the sets are groups of seed events of different physiological signals, and the sets include seed events spanning a same time span and can be indicative of a same sleep disordering event. Explained in a more visual way, the sets 12 are 'vertical' groups of seed events, whereas the coupling of seed events as described before is done 'horizontally'. The grouping of seed events into the set can include determining a substantially regular pattern in a co-occurrence of the seed events of the set. As an example, a median duration or an onset of a seed event may be similar across different physiological signals. In Figure 3, such a set 12 of seed events has been indicated.

Fig. 4 shows a third schematic graph representing of a plurality of physiological signals over time. As in the previous Figures, the graphs represent a plurality of physiological signals spanning a common time span, including a saturation of oxygen (SpO₂) signal 1, a PAT signal 2, a pulse rate signal 3 and a limb activity signal 4. In the SpO₂ signal 1, five desaturations of oxygen 7, 7a can be distinguished, which can be selected as being five seed events. In prior art method, an amplitude of these desaturations would be determined and compared to threshold values in order to decide whether or not these events may be associated with sleep disordering events. In the present graph, the first desaturation of oxygen 7a would probably be disqualified given the small difference of only 2% with respect to the previous baseline. However, in the method according to the invention, the substantially regular pattern in for example the onset of the desaturation, and/or in the duration of the seed event, would cause the method to detect also that sleep disordering event, for example an apnea, in spite of a relatively small oxygen desaturation value. Moreover, the step of grouping into sets of seed events between different physiological signals would strengthen a level of confidence of this detection since there is a substantially regular pattern in a co-occurrence of the seed events of the set, in particular a PAT signal amplitude drop 8a from a baseline, a pulse rate signal amplitude increase 9a from a baseline, and an increase in limb activity 10a, which all temporally align according to said substantially regular pattern.

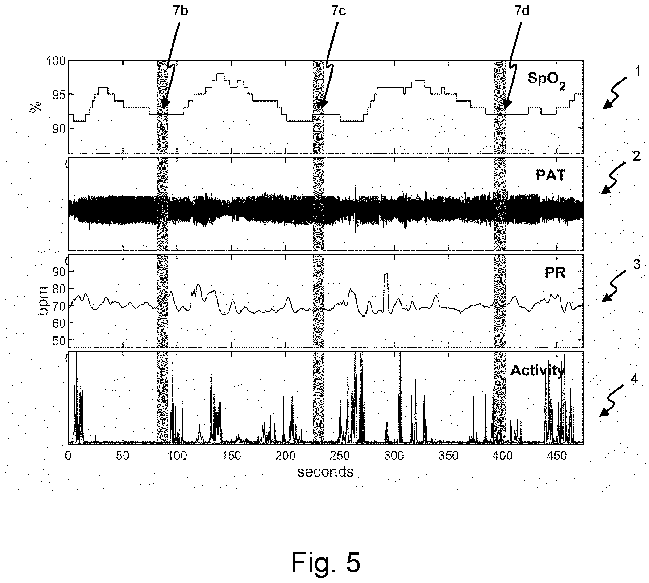

Fig. 5 shows a fourth schematic graph representing of a plurality of physiological signals 1, 2, 3, 4 over time. Again, in the SpO₂ signal 1, three desaturations of oxygen 7b, 7c, 7d can be distinguished, which can be selected as being three seed events being potentially indicatory of sleep disordering events. In prior art method, an amplitude of these desaturations would be determined and compared to threshold values. All oxygen desaturation events 7c,7d, and 7b would exceed the threshold value, so the method would decide that a sleep disordering event has been detected. Following the method of the present invention, substantially regular patterns would have to be determined within at least part of the seed events. This would prove to be difficult since even the oxygen desaturation signal 1, it is difficult to recognize a pattern, every desaturation 7b, 7c, 7d having a different overall shape. In the other physiological signals 2, 3 and 4, the situation is still more convincing since there does not seem to be any substantially regular pattern in spite of some PAT amplitude decreases, pulse rate spikes or limb activity spikes. The present method would therefore decide that no sleep disordering event has been detected, and that the desaturations may have been artefacts and/or due to another cause than a sleep disordering event. In a preferred embodiment of the method, the method can further comprise a step of feedback on a detection of a sleep disordering event or not by a selection of at least one seed event. In the present case, the method could indicate the reason why a seed event had not been selected, for example indicating "absence of substantially regular patterns". The feedback can preferably be rendered in natural language. Positive feedback could for example include "detection based on high degree of fit with a regular pattern in spite of low desaturation", which might apply to the physiological signals described in Figure 4.

As has been shown in the previous example, the present method for detecting sleep disordering events can improve accuracy of the detection of sleep disordering events, either based on a single, or a plurality of, physiological signals in that the method relies on contextual information around a single seed event in the form of determining substantially regular patterns and determining degrees of fit with said substantially regular patterns rather than following a decision tree based on threshold values for isolated seed events, as is done in prior art methods.

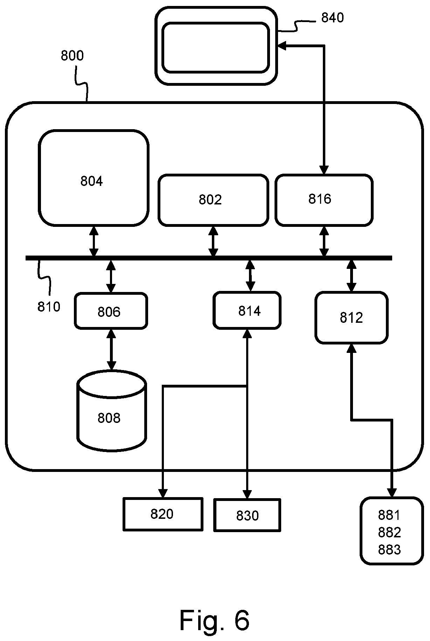

Fig. 6 shows a suitable computing system 800 comprising circuitry enabling the performance of steps according to the described embodiments. Computing system 800 may in general be formed as a suitable general-purpose computer and comprise a bus 810, a processor 802, a local memory 804, one or more optional input interfaces 814, one or more optional output interfaces 816, a communication interface 812, a storage element interface 806, and one or more storage elements 808. Bus 810 may comprise one or more conductors that permit communication among the components of the computing system 800. Processor 802 may include any type of conventional processor or microprocessor that interprets and executes programming instructions. Local memory 804 may include a random-access memory (RAM) or another type of dynamic storage device that stores information and instructions for execution by processor 802 and/or a read only memory (ROM) or another type of static storage device that stores static information and instructions for use by processor 802. Input interface 814 may comprise one or more conventional mechanisms that permit an operator or user to input information to the computing device 800, such as a keyboard 820, a mouse 830, a pen, voice recognition and/or biometric mechanisms, a camera, etc. Output interface 816 may comprise one or more conventional mechanisms that output information to the operator or user, such as a display 840, etc. Communication interface 812 may comprise any transceiver-like mechanism such as for example one or more Ethernet interfaces that enables computing system 800 to communicate with other devices and/or systems, for example with other computing devices 881, 882, 883. The communication interface 812 of computing system 800 may be connected to such another computing system by means of a local area network (LAN) or a wide area network (WAN) such as for example the internet. Storage element interface 806 may comprise a storage interface such as for example a Serial Advanced Technology Attachment (SATA) interface or a Small Computer System Interface (SCSI) for connecting bus 810 to one or more storage elements 808, such as one or more local disks, for example SATA disk drives, and control the reading and writing of data to and/or from these storage elements 808. Although the storage element(s) 808 above is/are described as a local disk, in general any other suitable computer-readable media such as a removable magnetic disk, optical storage media such as a CD or DVD, - ROM disk, solid state drives, flash memory cards, ... could be used.

As used in this application, the term "circuitry" may refer to one or more or all of the following:
(a) hardware-only circuit implementations such as implementations in only analog and/or digital circuitry and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions) and
(c) hardware circuit(s) and/or processor(s), such as microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in a server, a cellular network device, or other computing or network device.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A computer-implemented method for detecting sleep disordering events, the method comprising the steps of:
- obtaining at least one physiological signal;
- identifying from the at least one physiological signal seed events indicatory of sleep disordering events;
- determining substantially regular patterns within at least part of the seed events;
- determining degrees of fit of the seed events to the substantially regular patterns;
- detecting the sleep disordering events by selecting seed events based on the determined degrees of fit.

2. The method of claim 1, wherein the at least one physiological signal is at least one of a PAT signal, an oxygen saturation signal, a pulse rate signal, a respiratory effort signal, and an airflow signal.

3. The method of any of the preceding claims, wherein the seed events are at least one of a PAT signal amplitude drop from a baseline, a desaturation of oxygen, a pulse rate signal amplitude increase from a baseline, a respiratory effort signal amplitude decrease from a baseline, and an airflow signal amplitude decrease from a baseline.

4. The method of any of the preceding claims, wherein the substantially regular patterns includes a substantially regular morphological pattern.

5. The method of any of the preceding claims, wherein the substantially regular patterns includes a substantially regular temporal pattern.

6. The method of any of the preceding claims, wherein the step of determining the substantially regular patterns and the step of determining degrees of fit includes at least one of determining a duration of a seed event, determining an intensity of a seed event, deriving a morphological asymmetry and/or characterizing a morphological shape of a seed event.

7. The method of any of the preceding claims, wherein the step of determining the substantially regular patterns and the step of determining degrees of fit includes at least one of determining a starting point of the seed event, determining an end point of the seed event, determining a point of highest or lowest intensity and/or determining any other characteristic point of a seed event.

8. The method according to any of the preceding claims, wherein the step of detecting sleep disordering events includes using a classifier trained or developed for said detecting.

9. The method according to any of the preceding claims, wherein a plurality of physiological signals spanning a common time span which includes seed events is obtained.

10. The method according to claim 9, further comprising the step of grouping seed events from the plurality of physiological signals into sets, wherein the seed events grouped into the set are indicatory of the same sleep disordering event, wherein the grouping of seed events into the set includes determining a substantially regular pattern in a co-occurrence of the seed events of the set.

11. The method according to claim 8 and claim 10, wherein the classifier is configured to detect within the sets of seed events the respiratory related sleep disordering events, or the non-respiratory related sleep disordering events.

12. The method according to any of the preceding claims, further comprising a step of feedback on a detection of a sleep disordering event or not by a selection of at least one seed event, wherein the feedback is preferably rendered in natural language.

13. A controller comprising at least one processor and at least one memory including computer program code, the at least one memory and computer program code configured to, with the at least one processor, cause the controller to perform a method according to any of the preceding claims 1-12.

14. A computer program product comprising computer-executable instructions for performing the method according to any of the preceding claims 1-12 when the program is run on a computer.

15. A computer readable storage medium comprising computer-executable instructions for performing the method according to any of the preceding claims 1-12 when the program is run on a computer.
